# EUROPEAN PATENT SPECIFICATION

(11) **EP 3 330 546 B1**
(45) Date of publication and mention of the grant of the patent: **18.05.2022**
(21) Application number: 17211191.6
(22) Date of filing: 03.06.2005
(51) Int. Cl.: F04D 29/70, A01K 63/04, C02F 1/32

(54) **A PUMP UNIT**
PUMPENEINHEIT
UNITÉ DE POMPAGE

(30) Priority: 10.06.2004 GB 0412983; 23.12.2004 US 639711 P
(43) Date of publication of application: 06.06.2018
(62) Divisional of application: 05747227.6
(73) Proprietor: Exel Industries SA, 51200 Epernay (FR)
(72) Inventor: PATEL, Kaushik, Ishwarbhai, Northwood, Middlesex HA6 1QJ (GB); YUNG, Phong Yui, Popular, London E14 0SP (GB)
(74) Representative: CSY London

(56) References cited:
- EP-A1- 1 068 888
- EP-A2- 1 275 427
- DE-A1- 2 803 064
- DE-U1- 20 313 360
- GB-A- 2 352 796
- JP-A- 2000 296 391
- US-A- 5 160 607
- US-B1- 6 174 142
- US-B1- 6 423 218
- Matt Clark: "Practical Fishkeeping", Buyer's guide to pond pumps, 1 March 2002 (2002-03-01), pages 24-32, XP055532574,
- Anonymous: "Laguna product catalogue", Laguna, 1 January 2003 (2003-01-01), pages 1-12, XP055532593, Retrieved from the Internet: URL:http://www.hagen.com/pdf/ponds/Laguna- 2010.pdf [retrieved on 2018-12-11]

## Description

This invention relates to a pump unit for placing in a pond.

The water in ponds, such as ornamental ponds found in domestic gardens, can become, or give the appearance of being, dirty or polluted. For example, pond water may take on a green colour due to the growth of algae or similar organisms in that water. Pond water may also become dirtied with non-living solid matter.

To overcome this problem, pond filters are available for positioning near to a pond and for receiving a flow of water from the pond. The filters filter water received from the pond and then return the filtered water to the pond. Water is received from the pond along a hose running from a pump and is returned to the pond by way of another hose.

Ultra-violet (UV) filters are also available. These include a UV lamp for exposing water flowing through the UV filter to UV light. The UV filters are also for positioning near to a pond and may be positioned to receive water from a pump and to supply water to a pond filter of the kind just described.

JP 2000 296391 A describes an ultraviolet light sterilizer for use in water capable of sterilizing and disinfecting water.

EP 1 275 427 A2 describes a container provided with an internal pump producing cyclonic action and with filtering means to filter water in a body of water.

DE 28 03 064 A1 describes a pump used for swimming pool or aquarium cleaning having an axial delivery propeller screw rotor in a tube.

It is an object of this invention to improve the prior art.

According to a first aspect of this invention, there is provided a pond pump unit according to claim 1.

By providing a pump unit that includes both an electrical pump and a UV lamp, a neat and simple unit results that removes the need for a separate, unsightly UV filter situated near to the pond, together with the associated water hoses running to and from the pond. Instead, the UV lamp is placed out of sight in the pond.

The expression fountain outlet is used herein to mean a water outlet for creating a fountain.

The unit may be arranged such that the pump draws water from the pond past the UV lamp and then into an inlet of the pump unit. The unit may be arranged such that the pump draws water from the pond and pumps that water, from an outlet of the pump, past the UV lamp.

The unit may be arranged so that the pump and the lamp may be submerged in water. The unit may be arranged so that the unit may be submerged in water.

The unit may be arranged such that electrical components of the pump are contained in sealed container which is sealed against the ingress of water. The unit may be arranged such that electrical components of the lamp are contained in sealed container which is sealed against the ingress of water.

The unit may be arranged such that electrical components of the pump and of the lamp are contained together in a sealed container in the housing, the sealing being against the ingress of water. All of the electrical components of the pump and the lamp may be contained in the container. This allows the pump and the lamp to be powered by power from a single power lead to the container. Typically, the container is not of one-piece construction and may, instead, be formed from separable components. The sealed container may have a single power lead extending therefrom to supply electrical power to both of the pump and the lamp. The use of a single power lead, rather that a respective lead for each of the pump and the lamp simplifies construction of the unit and also simplifies installation of the unit in a pond. It may also be considered a safer configuration as, the fewer the number of power leads, the less is the likelihood of one being inadvertently damaged, for example by a lawn mower or other garden cutting apparatus, and conductors of the lead being thereby exposed.

A single sealed container is also advantageous in that it can require fewer seals than would, for example, two sealed containers.

The sealed container may be at least partly constituted by a removable cover for the UV lamp, the cover being removable from the remainder of the sealed container. The cover is preferably at least partly of a material, such as quartz, that tends to allow UV light to pass readily therethrough. Preferably the cover is a quartz tube, closed at one end and with mounting structure at the other, open, end for sealedly and releasably mounting the cover to the remainder of the container.

The unit may be arranged such that the major dimension of the UV lamp extends in the direction of water flow therepast. Preferably, where the UV lamp is tubular, the unit is arranged such that water flow past the lamp is substantially axial and, preferably, is all around the lamp in order to maximise exposure of the water to UV light emitted by the lamp.

The unit may also include mechanical filtering means and may be arranged such that the pump causes the water drawn from the pond to pass through those means such that the water is filtered thereby. The unit may be arranged such that the filtering means filters water drawn from the pond before or after that water reaches the pump and/or before or after that water flows past the lamp. The filtering means may include apertures formed in the housing of the unit and through which water from the pond is drawn by the pump. The apertures may be slots. The filtering means may include a filter member that is removably locatable in the housing. The filter member may include a mesh member. The mesh member may include a network of strands running in three dimensions between which water is drawn for filtering. The filter member may include a foam member for filtering water drawn therethrough. Preferably, the filtering means is arranged such that water is drawn from the pond therethrough before passing through the pump. This minimises the risk of the pump being clogged by solid matter or of the pump chopping up that matter into pieces. Chopping the matter into pieces is disadvantageous as the pieces may be small enough to avoid being caught by the mechanical filtering means.

The unit may include a bypass for allowing water drawn from the pond to bypass the filtering means when water flow though that means is hindered by at least partial clogging thereof. The bypass may include a valve. Preferably, the valve is arranged such that at least partial clogging of the filter means increases a pressure difference across the valve such that the valve opens and allows water drawn from the pond to pass therethrough and to thereby bypass at least part of the filter means. Where the filtering means includes a filter member, that member may include a bypass flow path therethrough in which the valve is situated. A bypass member may be provided that defines a bypass flow path with the valve situated therein. The valve may include a thin flap of material that is resiliently biased in a closed position and may be situated at an outlet side of the bypass flow path. The bypass member may be arranged to receive the filter member such that the bypass flow path extends through the member.

The housing may include a lid portion that is removably attachable to the remainder of the housing. The lid portion preferably is adjacent the filter medium such that removal of the lid portion facilitates access to the filter medium for inspection and/or removal thereof. The lid portion may include at least one catch for releasably attaching that portion to the remainder of the housing. The catch may be a sprung catch that is depressible by a user to allow removal of the lid portion.

The unit may include biological filtering structure arranged with a high surface area to volume ratio and arranged in the housing such that water drawn from the pond is caused to flow over and around the structure. Organisms present in the water will tend to grow on the surface of the structure and to clean water passing thereover and therearound. Preferably, the biological filtering structure is arranged in the housing such that water is drawn thereover and therearound after having passed through the filter means and before passing through the pump.

Preferably, the unit is arranged such that the fountain outlet receives water that has already flowed past the UV lamp. This is advantageous in that water which has been exposed to UV light from the lamp is then dispersed by the fountain outlet about the pond, rather than just in the vicinity of the unit. This tends to reduce the time taken for or, at least increase the likelihood of, substantially all of the water in the pond being drawn through the unit and being exposed to UV light from the lamp.

The unit may include a bypass outlet arranged to provide an outlet for at least some of the water drawn from the pond, so as to bypass the fountain outlet and rejoin to pond without forming a fountain. Preferably, the bypass outlet receives water that has already flowed past the lamp. The unit may include variable flow control means to variably control the flow of water though the bypass outlet. The variable flow control means may be arranged to be operable between a state in which flow through the bypass outlet is prevented and a state in which all water drawn from the pond is allowed to pass through the bypass outlet, and operable at states therebetween. Preferably, however, the variable flow control means is arranged so as to always allow at least some flow therethrough. This ensures that, should the fountain outlet become clogged, water may still exit the unit. The variable flow control means may be arranged to have the effect of controlling the height of the fountain from the fountain outlet. The variable flow control means may comprise a rotatable ring.

The bypass outlet may be positioned in the unit away from an inlet thereto through which water is drawn from the pond by the electrical pump. The bypass outlet may be positioned away from the mechanical filtering means, and preferably is positioned away from the apertures formed in the housing and through which water is drawn into the unit.

The housing may include outlet structure that at least partly defines the fountain outlet, the outlet structure being moveable relative to the remainder of the housing so as to allow water exiting the unit from the fountain outlet to be directed in a preferred direction. The outlet structure may be so moveable by the provision of a ball joint between the outlet structure and the remainder of the housing.

The unit may be arranged with a base portion and a projecting upper portion upstanding therefrom. The filtering means may conveniently be housed in the base portion, together, optionally, with the pump. The UV lamp may conveniently be housed in the upper portion, with the fountain outlet preferably arranged at the distal end of that portion.

The outlet structure may be removable and an obscuring plate may be provided in the housing to obscure viewing of the UV lamp when the outlet structure is removed.

The ball joint may form part of a ball joint assembly comprising the ball joint and the obscuring plate. The ball joint assembly may be captured in the housing. This can help prevent accidental viewing of the UV lamp. The obscuring plate may be dimensioned so that it is unable to pass through an aperture in the housing through which the ball joint projects. The obscuring plate may be mounted to the ball joint after the ball joint has been mounted in the housing. The obscuring plate may be fixed by a screw to the ball joint. Alternatively the obscuring plate may be clipped to or push fitted to the ball joint.

The unit may comprise a takeoff outlet for supplying water via a hose to a remote outlet. The remote outlet might be used to provide a waterfall or other water feature. The takeoff outlet may be distinct from any fountain or bypass outlet provided. The unit may comprise variable flow control means for variable control of water flow through the takeoff outlet. This variable flow control means may comprise a user operable rotatable ring. The rotatable ring may surround a portion of the housing and may be incomplete. This variable flow control means may comprise a collar control. Preferably the unit is arranged so that the takeoff outlet is positioned in the flow path so that water leaving via the takeoff outlet will have passed the UV lamp and preferably any other filter means included in the unit. The unit may comprise a takeoff outlet conduit providing a passage from the downstream end of the UV lamp to the takeoff outlet. The takeoff outlet conduit may comprise an inlet disposed at the downstream end of the UV lamp. The variable flow control means may comprise a closure member for closing, by controllable degrees, the inlet of the takeoff outlet conduit. The takeoff outlet may disposed towards the base of the unit. The takeoff outlet may be disposed closer to a base of the unit than is the distal end of the UV lamp. This can help with stability.

Where there are variable control means for the takeoff outlet and the fountain bypass outlet, the manually operated actuators, for example rotatable rings/control collars may be disposed adjacent one another. The bypass may be arranged so that water exiting via the bypass passes between the two control rings.

Specific pump units in which the invention is embodied are described now by way of example only and with reference to the accompanying drawings, in which:
Figure 1 is a perspective view of a first pump unit;
Figure 2 is a perspective view of the unit of Figure 1 with part of a housing thereof removed to show internal detail;
Figure 3 is a perspective view of mechanical filtering means of the pump unit of Figure 1;
Figure 4 is another perspective view of the pump unit of Figure 1;
Figure 5 is a sectional view of the pump unit of Figure 1;
Figure 6 is a sectional view of a second pump unit which includes a take off outlet; and
Figure 7 is an exploded view of part of the pump unit of Figure 6 which illustrates a flow control mechanism for the take off outlet.

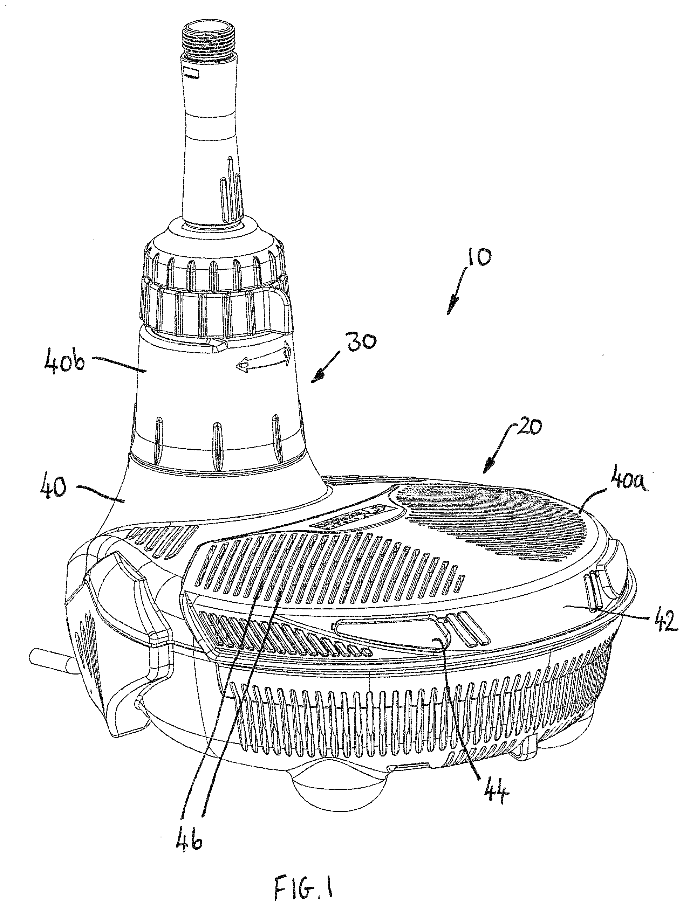

Figure 1 shows a pump unit 10 having a squat cylindrical base portion 20 and a narrower cylindrical upper portion 30 that projects from the base portion 20 so as to be upstanding from it. The upper portion 30 is positioned towards the periphery of the base portion 20. The pump unit 10 is for placing in a pond so as to be submerged in water of the pond.

The pump unit 10 has a plastic housing 40 that houses components (not shown in Figure 1) of the unit 10. The housing 40 is made up of a base housing 40a that is formed to define the outer shape of the base portion 20, and an upper housing 40b that is formed to define the outer shape of the upper portion 30. The majority of the upper part of the base housing 40a forms a removable lid 42 that is removable from the remainder of the base housing 40a. The lid 42 includes two catches (only part of which is visible at 44) that are for releasably securing the lid 42 to the remainder of the base housing 40a. Each of the catches has a respective button portion 44 that projects through an aperture formed in the lid 42 for operation by a user to disengage the respective catch from the remainder of the base housing 40a and thereby allow removal of the lid 42.

The base housing 40a is formed with many apertures through it in the form of slots 46. The slots 46 are formed in the lid 42 and in the side of the base housing 40a adjacent the lid 42.

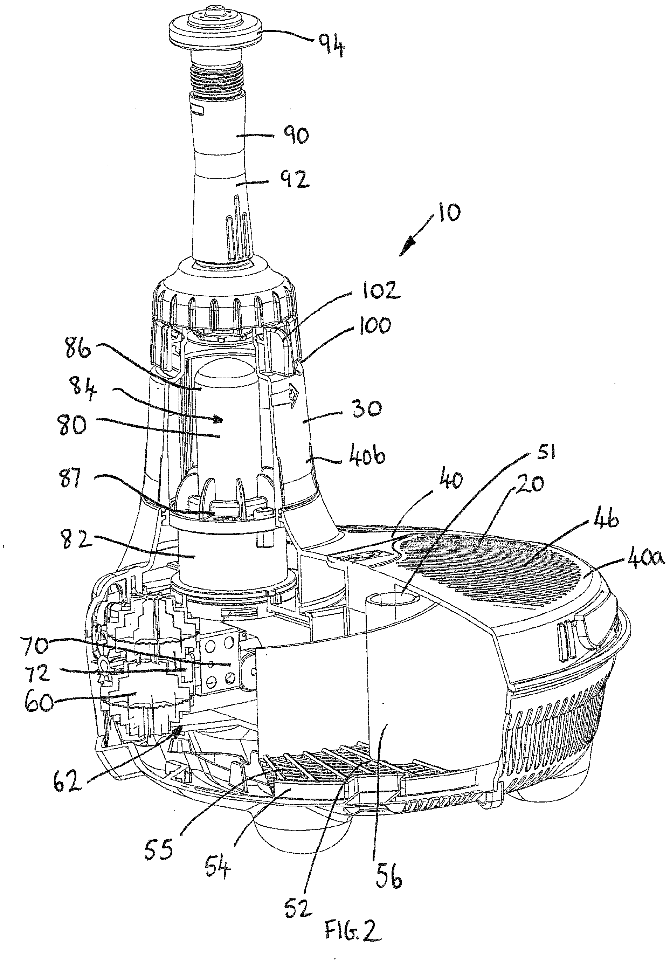

Figure 2 shows the pump unit 10 with part of the housing 40 removed in order to show the inside of the unit 10. With reference to Figure 2, the pump unit 10 includes mechanical filtering means in the form of a foam block 50, biological filtering structure in the form of "biomedia" 60, an electrical pump 70, a UV lamp 80, a fountain outlet 90 and a bypass outlet 100.

The foam block 50 is generally semi-circular in cross-section. The block 50 is arranged in the squat cylindrical base portion 20 against the outside of the base portion 20 and underneath the lid 42. The block 50 is shaped to substantially fill the inside of the unit 10 that is beneath the lid 42. The block 50 is made of a coarse foam-like material that is arranged to mechanically filter water passing through it by straining solid matter from that water.

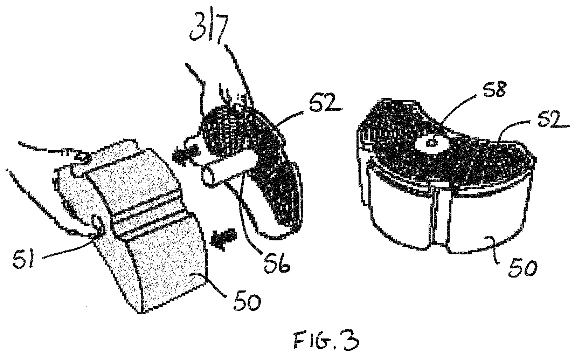

The foam block 50 is supported on a bypass member 52 on which the block 50 is seated. The bypass member 52 is formed of moulded plastic and includes a base portion 54 and a cylindrical tube 56. The base portion 54 is shaped as the base of the block 50 and includes many apertures 55 through it. The base portion 54 also includes a peripheral wall that follows snugly the sides of the block 50. The cylindrical tube 56 is upstanding from the base portion 54 and is accommodated in the block 50 by way of a cylindrical aperture 51 extending through the block 50, from its base to its top. The tube 56 extends through the aperture 51. The foam block 50 and the bypass member 52 are also shown in Figure 3. As shown in Figure 3, the lower end of the tube 56 is provided with a flap valve 58. The valve 58 is arranged to allow liquid flow in a downwards direction only, and only when a pressure differential across the valve (i.e. the difference between pressure at the inlet side of the valve and pressure at the outlet side of the valve) is above a certain pressure difference.

With reference to Figure 2, the unit 10 includes a chamber 62 in the base portion 20 that communicates with the underside of the base portion 54 of the bypass member 52. This chamber 62 is generally underneath and extends to each side of the upper portion 30. The chamber 62 accommodates the biomedia 60. The biomedia 60 are plastic structure with high surface area to volume ratio and, in this embodiment, take the form of six ball-like objects formed of many intersecting, jagged, generally planar surfaces.

The electrical pump 70 is positioned in the housing 40 so as to be in the chamber 62 in which the biomedia 60 are also accommodated. The electrical pump 70, however, is more centrally positioned so as to be underneath the upper portion 30. Thus, the biomedia 60 are positioned to the sides of the electrical pump 70. Casing 72 of the electrical pump 70 forms a barrier at the base of that upper portion 30 between the chamber 62 and the inside of the upper portion 30 of the unit 10. The casing 72 also defines an inlet of the electrical pump (not visible in Figure 2) that extends into the chamber 62 and an outlet (not visible in Figure 2) that extends into the upper portion 30. The electrical pump 70 may be arranged to receive a mains voltage at, for example, 240V AC or 110V AC; or a low voltage at, for example, 24V AC. In this embodiment, the electrical pump 70 operates at a mains voltage.

The UV lamp 80 is positioned inside the upper portion 30 of the pump unit 10 and is an arrangement of several parts. A base 82 of the lamp 80 is joined to the pump casing 72 so as to be an extension of that casing 72 and so as to project therefrom upwards into the upper portion 30. The base 82 houses an electrical socket (not shown in Figure 2) into which a UV bulb 84 of the UV lamp 80 is releasably locatable. The lamp 80 also includes a removable cover in the form of a quartz tube 86, closed at one end and fitted with a bayonet fitting 87 at the other. The bayonet fitting 87 and the base 82 are such that the fitting 87 can be received in the base 82 and twisted into a position in which a watertight seal is created between the base 82 and the fitting 87, and hence also the quartz tube 86. A screw 88 is provided for keeping the bayonet fitting 87, and hence the quartz tube 86, in place. Thus, the quartz tube 86, with the UV bulb 84 inside, project upwardly from the base 82 into and through the upper portion 30 of the unit 10.

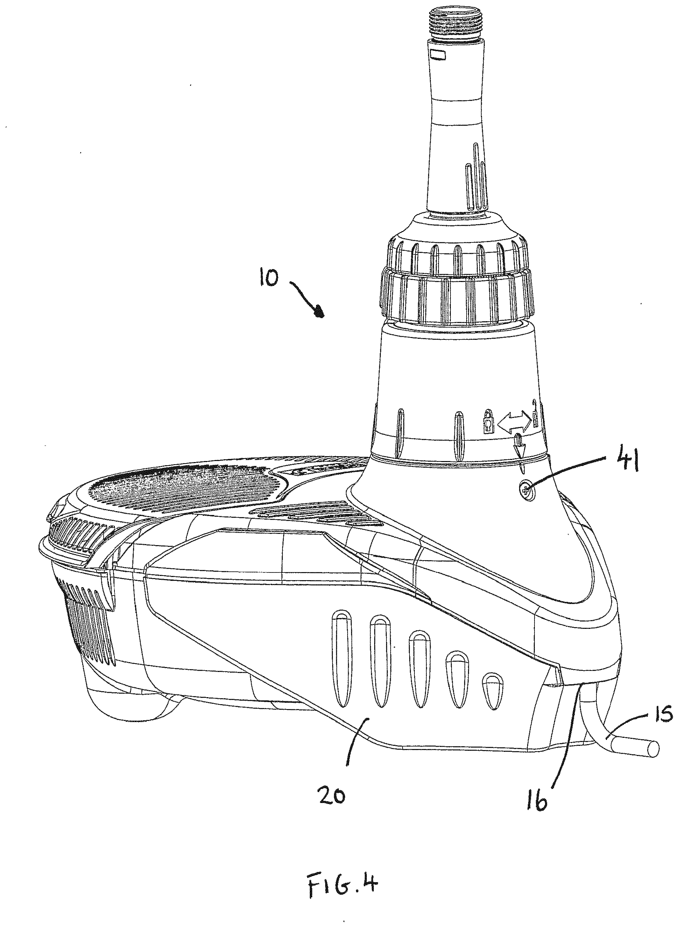

Taken together: the casing 72 of the pump 70; and the base 82, bayonet fitting 87 and quartz tube 86 of the UV lamp 80, form a sealed container in which all the electrical components of the pump unit 10 are housed in a watertight fashion. This allows the pump unit 10 to have a single electrical power lead, shown at 15 in Figure 4 running thereto. The power lead 15 enters the base portion 20 of the unit 10 via a suitable aperture 16, and from there enters the sealed container via an aperture (not shown) in the casing 72 of the pump 70.

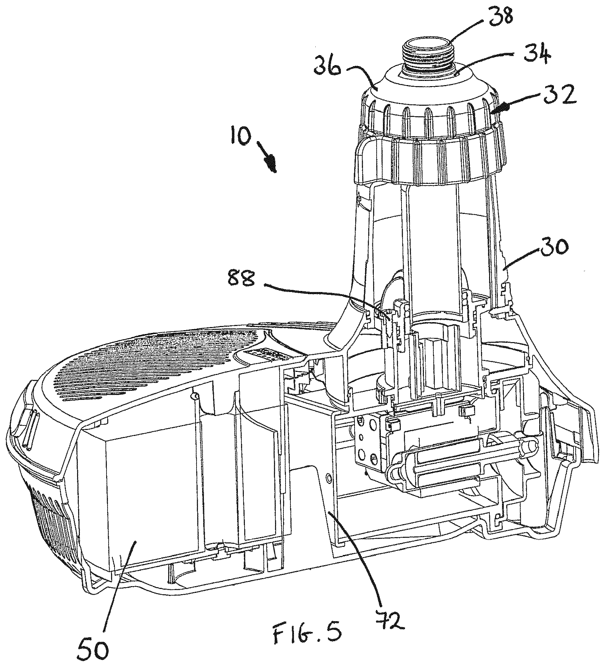

With reference to Figure 5, the upper end of the upper portion 30 of the pump unit 10 is formed into a socket 32 for receiving a ball joint 34. The ball joint 34 is a short length of plastic tube with the mid portion of its outer surface formed to resemble the outer surface of a sphere. A removable threaded collar 36 is provided for releasably and variably securing the ball joint 34 to the upper end of the upper portion 30, that upper end being formed with a cooperating thread. When secured in this way, an upper end 38 of the ball joint 34 projects beyond the collar 36. The upper end 38 of the ball joint 34 is threaded.

A fountain outlet 90 is provided for attachment to the threaded upper end 38 of the ball joint 34. This outlet 90 is shown attached in Figure 2. The fountain outlet 90 includes a pipe 92 with a nozzle portion 94 at one end. The other end of the pipe 92 is threaded and is for cooperating with the threaded upper end 38 of the ball joint 34 for releasable attachment to that end 38. The nozzle portion 94 is formed with holes therethrough arranged to direct water pumped therethrough into a jet of one or more streams.

The upper portion 30 of pump unit 10 includes a bypass outlet 100 towards its upper end, but below the fountain outlet 90, the ball joint 34 and the collar 36. The bypass outlet 100 includes an outlet aperture (not shown) in the upper casing 40b of the upper portion 30 that allows communication between the inside of the upper portion 30 and the outside. The bypass outlet 100 also includes a rotatable ring 102 that surrounds the upper portion 30 in the region of the outlet aperture. This region of the upper portion 30 is of reduced diameter, such that the ring 102 can be accommodated around that region so as to be flush with the surrounding outside surface of the upper portion 30. The ring 102 is formed and fitted to the upper portion 30 so as to be rotatable between a position in which the outlet aperture is almost closed by the ring 102 and a position in which the outlet aperture is open, and rotatable to positions between these two extremes.

For operation, the pump unit 10 is placed in a pond, such as a pond in the garden of a house. The pump unit 10 is positioned in the pond such that the outlet aperture in the upper portion 30 of the unit 10 is below the pond's water line and such that the nozzle 94 is above the water line. The electrical lead 15 is then connected, in this embodiment, to a mains source of electrical power. This causes the unit 10 to operate.

In operation, the electrical pump 70 and the UV lamp 80 receive power via the lead 15 and operate. The pump 70 reduces the pressure in its inlet below the ambient pressure of pond water surrounding the pump unit 10. This causes pond water to be drawn towards and into the inlet. Thus, water from the pond is drawn through the slots 46 in the base housing 40a, through the foam block 50 and the apertures 55 of the base portion 54 on which the block 50 sits, into the chamber 62 in the base portion 20 and, from there, into the pump 70 via its inlet. As the water is drawn along this flow path, the slots 46 in the base housing 40a serve to strain large pieces of solid matter from the water as it is drawn therethrough; and the foam serves to subsequently strain finer pieces of solid matter from the water. In the chamber 62, the water is drawn over and around the many surfaces of the biomedia 60 before entering the pump 70. Micro-organisms on the surfaces of the biomedia 60 tend to operate on the water to clean it further by feeding on ammonia and converting it into nitrite and then nitrate.

Water that passes into the operating electrical pump 70 exits the pump 70 via its outlet into the upper portion 30 of the pump unit 10. The pump 70 increases the pressure of water at its outlet to a pressure above the ambient pressure of water in the pond. Thus, water flows from the outlet, up the upper portion 30, around and past the operating UV lamp 80 to the upper end of the upper portion 30. From here, some of the water flows through the ball joint 34, up the pipe 92 of the fountain outlet 90 to exit the unit 10 in one or more jets via the nozzle 94, thereby forming a fountain of water before returning to the pond. The remainder of the water flows through the aperture of the bypass outlet 100 to thereby exit the unit 10 and rejoin the remainder of the water in the pond. As the water flows around and past the UV lamp 80, that lamp 80 exposes the water and its contents to UV light. The UV light tends to cause the cell walls of algae in the water to rupture, which in turn causes the algae to gather together in groups that either sink to the bottom of the pond or are caught by mechanical filtering.

The rotatable ring 102 of the bypass outlet 100 is rotatable to vary the amount of water that exits the pump unit 10 via that bypass outlet 100 so as to bypass the fountain outlet 90. Rotating the ring 102 to open the bypass outlet 100 to its fullest extent results in substantially all of the water from the pump 70 exiting the unit 10 via the bypass outlet 90. This feature may be used when only a small fountain of water is required. Alternatively, the ring 102 can be rotated to close the bypass outlet 100 to its fullest extent. This, however, does not completely close the bypass outlet 100, but still allows some water from the pump 70 to exit the unit 10 therethrough. This ensures that water can still flow through the unit 10 even if the nozzle 94 of the fountain outlet 90 were to become blocked. Selectively rotating the ring 102 to positions between these two extremes allows the height of the fountain of water produced by the unit 10 to be varied in a controlled manner.

In the event that the foam block 50 becomes clogged by solid matter trapped therein, an increased pressure differential is created across the valve 58 in tube 56 of the bypass member 52 on which the block 50 sits. This causes the valve 58 to open and to allow water to flow along the tube 56 and into the chamber 62 and the pump 70 in the base portion 20. Thus, the foam block 50 is bypassed when clogged. Again, this allows the pump unit 10 to continue to operate and, in particular, to continue to expose pond water to the UV lamp 80 when the foam block 50 is at least partially clogged.

Should the foam block 50 become blocked in the way described, it can be removed and either cleaned or replaced. To remove the foam block 50, the lid 42 of the base portion 20 is first removed from the remainder of that portion 20 by pressing on the two button portions 44 of the catches, and lifting the lid 42. This allows access to the foam block 50. The foam block 50, together with the bypass member 52 on which the foam block 50 sits, can then be removed from the base portion 20. The foam block 50 and the bypass member 52 would then be separated from one another in order to allow cleaning or replacement of the foam block 50. The pump unit 10 is reassembled by carrying out this process in reverse.

The UV bulb 84 may also be removed for, for example, replacement. It this is to be done, electrical power to the pump unit 10 should first be cut off. The unit 10 may then be removed from the pond. Access to the UV lamp 80 is gained by removing the upper housing 40b around the upper portion 30 of the unit 10. This part of the housing 40 is twist mountable onto the remainder of the housing 40 (i.e. the base housing 40a) and is easily removed once a locating screw (shown at 41 in Figure 4) is removed. The quartz tube 86 can then be removed by unscrewing the screw 88 in the bayonet fitting 87 and twisting that fitting 87. The UV bulb 84 can then be removed, replaced and the process reversed to re-assemble the pump unit 10.

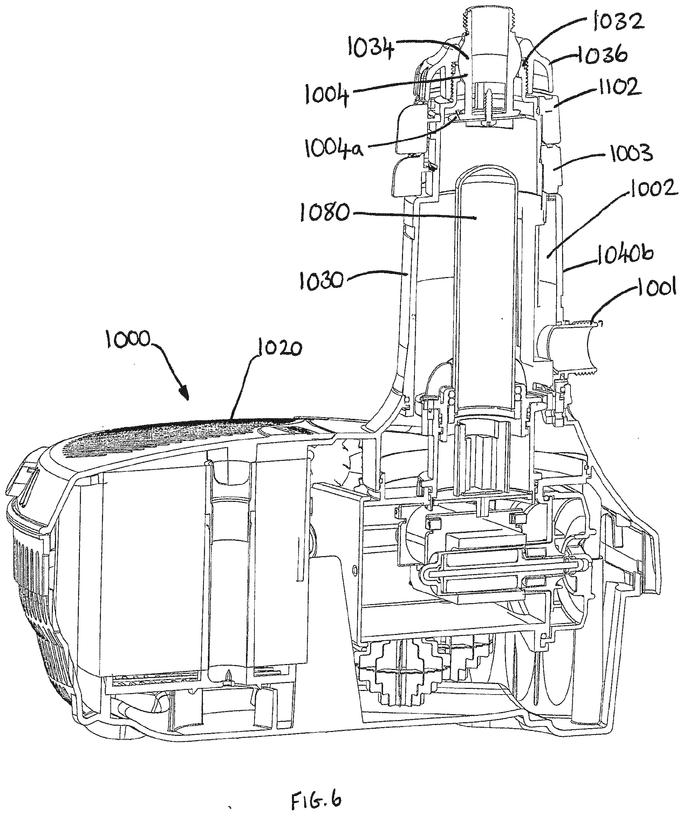

Figure 6 shows a pump unit 1000 which is similar to the pump unit 10 shown in Figures 1 to 5. The pump unit 1000 shown in Figure 6 is arranged for use in a larger pond than that shown in Figure 5 but in many respects is the same, or substantially the same, as the pond unit 10 shown in Figures 1 to 5. Therefore, detailed description of the majority of the pump unit 1000 shown in Figure 6 will be omitted and the same reference numerals are used to refer to the corresponding parts with the exception that 1000 is added to each reference numeral so that 20 becomes 1020 and so on.

The base portion 1020 of the pump unit 1000 shown in Figure 6 is basically the same as that shown in Figures 1 to 5 except that it has increased dimensions. Again this base portion 1020 is arranged to stand submerged in the water of a pond. Indeed, as is the case for the pump unit shown in Figures 1 to 5, the whole unit is arranged so as to be submersible in water with the tip of a fountain outlet (not shown in Figure 6) at or near the surface.

In the pump unit 1000 shown in Figure 6 there is again the cylindrical upper portion 1030 which is narrower than the squat cylindrical base portion 1020. In this pump unit 1000 a take off outlet 1001 is provided in the upper portion 1030. This take off outlet 1001 is arranged to allow a supply of filtered water to leave the pump unit 1000 via a suitable hose connected to the take off outlet 1001 and to be fed by the hose to a remote outlet, for example so as to form a waterfall feature.

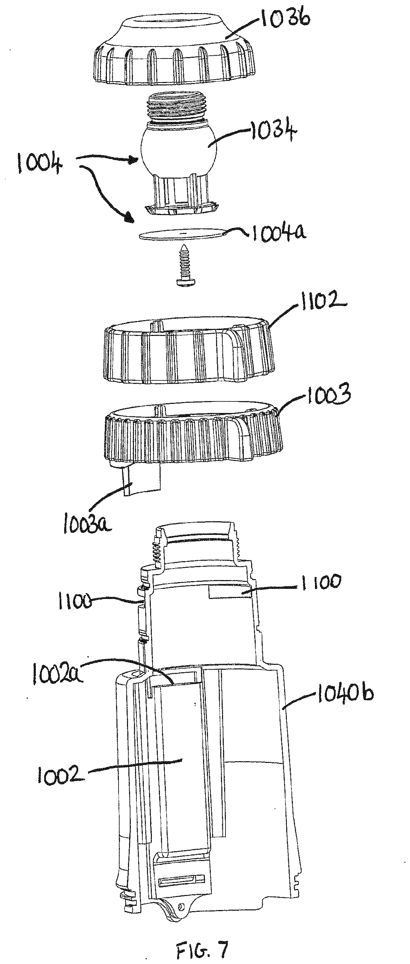

The pump unit 1000 also includes a flow control for controlling the flow which leaves the pump unit 1000 via the take off outlet 1001. As most clearly seen by reference to Figure 7 as well as Figure 6, a take off outlet conduit 1002 is provided on the inside surface of the upper housing 1040b of the upper portion 1030. This conduit 1002 has an inlet 1002a towards its upper end and feeds into the take off outlet 1001 at its lower end. By reference to Figure 6 it can be seen that the inlet 1002a to the conduit 1002 is at the downstream end of the UV lamp 1080 so that water entering the conduit 1002 to leave the pump unit 1000 via the take off outlet 1001 will have passed the UV lamp 1080.

In this pump unit 1000, as well as a rotatable ring or collar control 1102 for controlling a by-pass outlet 1100 for allowing water to by-pass the fountain outlet 1090 (in the same way as in the first pump unit shown in Figures 1 to 5), there is provided a second rotatable ring or collar control 1003 which is operable by a user to control the flow allowed out of the take off outlet 1001. The collar control 1003 for the take off outlet 1001 comprises a depending tongue 1003a which is best seen in Figure 7 and which is arranged to substantially blank off the inlet 1002a to the conduit 1002 if the collar 1003 has a rotational position which is such that the tongue 1003a is in register with the outlet 1002a. As can be seen by consideration of Figure 7, if the collar 1003 is moved away from register with the inlet 1002a, the inlet 1002a will be progressively opened until the tongue 1003 moves completely out of the way of the inlet 1002a.

Generally speaking in pump units of the type considered in this application it is not necessary to obtain a perfect seal between components such as the tongue 1003a and the opening 1002a to the conduit 1002. If some water is able to leak out through such a path this is not of great concern. What is desirable is the ability to control, at a general level, the water flow which is occurring.

It can be seen that the conduit 1002 is defined partially by the side wall of the housing 1040b and partially by a component which is mounted to that side wall. The conduit extends in a generally axial direction relative to the upper portion 1030 and also extends generally axially relative to the UV lamp 1080. The arrangement of the conduit 1002 is such that the water flow path passes the UV lamp in a first direction and then passes back along the conduit 1002 in an opposite direction before leaving via the take off outlet 1001. It will be noted that in this embodiment a confined conduit 1002 is provided which extends around only a relatively small part of the circumference of the housing 1040 of the upper portion 1030. In an alternative, an annular conduit could be provided by providing a cylindrical wall portion which fits inside the housing 1040b and which has one or more suitable inlets at its upper end to allow the ingress of water.

Fountain by-pass outlets 1100 of the second pump unit 1000 can also be seen in Figure 7, and as in the first pump unit 10, water flow through these is controlled by the respective collar control 1102. Rotation of this collar control 1102 serves to alter the proportion of the by-pass outlets 1100 which are blocked to water flow.

The pump unit 1000 shown in Figures 6 and 7 also comprises a modified form of ball joint assembly 1004 which includes a ball joint 1034 which is the same as that in the pump unit of Figures 1 to 5 and which is again received in a socket 1032 in the upper portion 1030 of the unit. In this unit however, a UV lamp obscuring disc 1004a is attached to the ball joint 1034 using a screw. This UV lamp obscuring disc 1004a serves to substantially prevent viewing of the UV lamp 1080 if the retaining collar 1036 is removed from the upper portion of the unit 1030. Furthermore, the inclusion of this obscuring disc 1004a also serves to capture the ball joint assembly 1004 in the upper portion of the unit. As can be best seen in Figure 6, the socket portion 1032 prevents movement of the ball joint assembly 1004 in one direction whereas the obscuring disc 1004a prevents movement of the assembly 1004 in the opposite direction. This means that not only does the obscuring disc 1004a blank out a possible line of sight to the UV lamp 1080 when the retaining collar 1036 is removed but also prevents removal of the ball joint assembly 1004 from the external side of the casing 1040b without first removing the obscuring disc 1004a.

Whilst in this embodiment the obscuring disc 1004a is fixed to the ball joint 1034 using a screw, in alternatives the obscuring disc may be push fitted or clipped onto the ball joint 1034.

## Claims

1. A pond pump unit (10) for placing in a pond, the unit comprising a housing (40) in which is housed an electrical pump (70) and an electrical UV lamp (80), wherein the unit is arranged such that the pump can draw water from the pond and is operable to cause that water to flow past the UV lamp, so as to be exposed to UV light therefrom, before rejoining the pond **characterised in that** the pump unit further comprises a fountain outlet (90) for creating a fountain.

2. A pond pump unit according to claim 1 in which the electrical components of the pump (70) are contained in a sealed container (72) which is sealed against the ingress of water and the electrical components of the lamp are contained in a sealed container (82, 86, 87) which is sealed against the ingress of water.

3. A pond pump unit according to claim 1 wherein the electrical components of the pump (70) and the UV lamp (80) are contained together in a sealed container (72, 82, 86, 87) in the housing, the sealing of the sealed container being against the ingress of water.

4. A pond pump unit according to any preceding claim arranged such that the major dimension of the UV lamp (80) extends in the direction of water flow therepast.

5. A pond pump unit according to any preceding claim arranged such that the water flow is all around the lamp (80) in order to maximise exposure of the water to

6. A pond pump unit according to any preceding claim, which further comprises a mechanical filtering means (46, 50, 55) and is arranged such that the pump causes the water drawn from the pond to pass through those means such that the water is filtered thereby.

7. A pond pump unit according to claim 6 wherein the filtering means comprises apertures (46) formed in the housing (40) of the unit and through which water from the pond is drawn by the pump.

8. A pond pump unit according to claim 6 or claim 7 wherein the filtering means include a filter member (50) that is removably locatable in the housing.

9. A pond pump unit according to any one of claims 6 to 8 which further comprises a bypass (52) for allowing water drawn from the pond to bypass at least part of the filtering means when water flow though that means is hindered by at least partial clogging thereof.

10. A pond pump unit according to any preceeding claim which further comprises biological filtering structure (60) arranged with a high surface area to volume ratio and arranged in the housing (40) such that water drawn from the pond is caused to flow over and around the structure.

11. A pump unit according to any preceding claim in which the fountain outlet comprises a pipe (92) with a nozzle portion (94) at one end for creating the fountain, wherein the nozzle portion (94) is formed with holes therethrough arranged to direct water pumped therethrough into a jet of one or more streams.

12. A pond pump unit according to any preceding claim which further comprises a bypass outlet (100) arranged to provide an outlet for at least some of the water drawn from the pond, so as to bypass the fountain outlet and rejoin to the pond without forming a fountain.

13. A pond pump unit according to claim 12 in which the unit is arranged so that the bypass outlet (100) receives water that has already flowed past the UV lamp (80).

14. A pond pump unit according to claim 12 or claim 13 which further comprises a variable flow control means to variably control the flow of water though the bypass outlet (100) and/or the fountain outlet (90).

15. A pond pump unit according to claim 14 wherein the variable flow control is arranged to be operable between a state in which flow through the bypass outlet (100) is prevented and a state in which all water drawn from the pond is allowed to pass through the bypass outlet, and is operable at states therebetween.

16. A pond pump unit according to claim 14 or claim 15 in which the variable flow control is user adjustable by means of a rotatable collar (102).

17. A pond pump unit according to any one of claims 13 to 16 wherein the bypass outlet (100) is positioned in the unit (30) away from an inlet thereto through which water is drawn from the pond by the electrical pump.

18. A pond pump unit according to any preceding claim in which the unit comprises a takeoff outlet (1001) for supplying water via a hose to a remote outlet.

19. A pond pump unit according to claim 18 in which the unit comprises variable flow control means (1102) for variable control of water flow through the takeoff outlet.

20. A pond pump unit according to any one of claims 18 to 19 in which the unit is arranged so that the takeoff outlet is positioned in the flow path so that water leaving via the takeoff outlet will have passed the UV lamp.

21. A pond pump unit according to any preceding claim which is arranged to be submersible in water.

22. A pond pump unit according to any preceding claim arranged with a base portion (20) and a projecting upper portion (30) upstanding therefrom.

23. A pond pump unit according to any preceding claim which is a domestic garden pond pump unit.

## Patentansprüche

1. Teichpumpeneinheit (10) zum Stellen in einen Teich, wobei die Einheit ein Gehäuse (40) aufweist, in dem eine elektrische Pumpe (70) und eine elektrische UV-Lampe (80) untergebracht sind, wobei die Einheit so angeordnet ist, dass die Pumpe Wasser aus dem Teich saugen kann, und funktionell ist, um zu veranlassen, dass dieses Wasser an der UV-Lampe vorbeifließt, damit es UV-Licht von ihr ausgesetzt wird, bevor es wieder in den Teich zurückkehrt, **dadurch gekennzeichnet, dass** die Pumpeneinheit ferner einen Springbrunnenauslass (90) aufweist, um einen Springbrunnen zu erzeugen.

2. Teichpumpeneinheit nach Anspruch 1, bei der die elektrischen Bauteile der Pumpe (70) in einem abgedichteten Behälter (72) enthalten sind, der gegen das Eindringen von Wasser abgedichtet ist, und die elektrischen Bauteile der Lampe in einem abgedichteten Behälter (82, 86, 87) enthalten sind, der gegen das Eindringen von Wasser abgedichtet ist.

3. Teichpumpeneinheit nach Anspruch 1, wobei die elektrischen Bauteile der Pumpe (70) und der UV-Lampe (80) zusammen in einem abgedichteten Behälter (72, 82, 86, 87) in dem Gehäuse enthalten sind, wobei die Abdichtung des abgedichteten Behälters gegen das Eindringen von Wasser ist.

4. Teichpumpeneinheit nach einem der vorhergehenden Ansprüche, die so angeordnet ist, dass die Hauptabmessung der UV-Lampe (80) sich in der Richtung des Wasserflusses an ihr vorbei erstreckt.

5. Teichpumpeneinheit nach einem der vorhergehenden Ansprüche, die so angeordnet ist, dass der Wasserfluss um die ganze Lampe (80) herum erfolgt, um die Bestrahlung des Wassers mit von der Lampe ausgestrahltem UV-Licht zu maximieren.

6. Teichpumpeneinheit nach einem der vorhergehenden Ansprüche, die ferner ein mechanisches Filtermittel (46, 50, 55) aufweist und so angeordnet ist, dass die Pumpe das aus dem Teich gesaugte Wasser durch diese Mittel strömen lässt, so dass das Wasser dadurch gefiltert wird.

7. Teichpumpeneinheit nach Anspruch 6, wobei das Filtermittel Öffnungen (46) aufweist, die in dem Gehäuse (40) der Einheit gebildet sind und durch die von der Pumpe Wasser aus dem Teich gesaugt wird.

8. Teichpumpeneinheit nach Anspruch 6 oder Anspruch 7, wobei das Filtermittel ein Filterelement (50) beinhaltet, das entfernbar in das Gehäuse eingesetzt werden kann.

9. Teichpumpeneinheit nach einem der Ansprüche 6 bis 8, die ferner eine Umleitung (52) aufweist, um aus dem Teich gesaugtes Wasser zumindest einen Teil des Filtermittels umgehen zu lassen, wenn Wasserdurchfluss durch dieses Mittel durch zumindest teilweise Verstopfung von ihm behindert wird.

10. Teichpumpeneinheit nach einem der vorhergehenden Ansprüche, die ferner eine biologische Filterstruktur (60) aufweist, die mit einem hohen Verhältnis von Oberfläche zu Volumen angeordnet ist und in dem Gehäuse (40) angeordnet ist, so dass veranlasst wird, dass aus dem Teich gesaugtes Wasser über und um die Struktur fließt.

11. Pumpeneinheit nach einem der vorhergehenden Ansprüche, bei der der Springbrunnenauslass ein Rohr (92) mit einem Düsenteil (94) an einem Ende zum Erzeugen des Springbrunnens aufweist, wobei der Düsenteil (94) mit durch ihn hindurchgehenden Löchern versehen ist, die so angeordnet sind, dass sie durch sie hindurchgepumptes Wasser in einen Strahl aus einem oder mehreren Strömen lenken.

12. Teichpumpeneinheit nach einem der vorhergehenden Ansprüche, die ferner einen Umleitungsauslass (100) aufweist, der angeordnet ist, um einen Auslass für zumindest einen Teil des aus dem Teich gesaugten Wassers bereitzustellen, um den Springbrunnenauslass zu umgehen und zum Teich zurückzukehren, ohne einen Springbrunnen zu bilden.

13. Teichpumpeneinheit nach Anspruch 12, bei der die Einheit so angeordnet ist, dass der Umleitungsauslass (100) Wasser erhält, das bereits an der UV-Lampe (80) vorbeigeflossen ist.

14. Teichpumpeneinheit nach Anspruch 12 oder Anspruch 13, die ferner ein verstellbares Durchflusssteuermittel zum verstellbaren Steuern des Wasserdurchflusses durch den Umleitungsauslass (100) und/oder den Springbrunnenauslass (90) aufweist.

15. Teichpumpeneinheit nach Anspruch 14, wobei die verstellbare Durchflusssteuerung angeordnet ist, um zwischen einem Zustand, in dem der Durchfluss durch den Umleitungsauslass (100) verhindert wird, und einem Zustand, in dem sämtliches aus dem Teich gesaugtes Wasser durch den Umleitungsauslass hindurchfließen darf, funktionsfähig zu sein, und bei dazwischenliegenden Zuständen funktionsfähig ist.

16. Teichpumpeneinheit nach Anspruch 14 oder Anspruch 15, bei der die verstellbare Durchflusssteuerung mithilfe eines drehbaren Rings (102) vom Benutzer verstellbar ist.

17. Teichpumpeneinheit nach einem der Ansprüche 13 bis 16, wobei der Umleitungsauslass (100) in der Einheit (30) von einem Einlass in diese, durch den von der elektrischen Pumpe Wasser aus dem Teich gesaugt wird, entfernt positioniert ist.

18. Teichpumpeneinheit nach einem der vorhergehenden Ansprüche, bei der die Einheit einen Entnahmeauslass (1001) zum Zuführen von Wasser über einen Schlauch zu einem abgesetzten Auslass aufweist.

19. Teichpumpeneinheit nach Anspruch 18, bei der die Einheit ein verstellbares Durchflusssteuermittel (1102) zum verstellbaren Steuern des Wasserdurchflusses durch den Entnahmeauslass aufweist.

20. Teichpumpeneinheit nach einem der Ansprüche 18 bis 19, bei der die Einheit so angeordnet ist, dass der Entnahmeauslass im Durchflussweg positioniert ist, so dass über den Entnahmeauslass austretendes Wasser an der UV-Lampe vorbeigeflossen ist.

21. Teichpumpeneinheit nach einem der vorhergehenden Ansprüche, die angeordnet ist, um in Wasser untergetaucht sein zu können.

22. Teichpumpeneinheit nach einem der vorhergehenden Ansprüche, die mit einem Unterteil (20) und einem davon aufragenden vorstehenden Oberteil (30) angeordnet ist.

23. Teichpumpeneinheit nach einem der vorhergehenden Ansprüche, die eine Gartenteichpumpeneinheit für den Hausgebrauch ist.

## Revendications

1. Unité formant pompe pour bassin (10) destinée à des fins de mise en place dans un bassin, l'unité comportant un logement (40), dans lequel est logée une pompe électrique (70), et une lampe UV électrique (80), dans laquelle l'unité est agencée de telle sorte que la pompe peut aspirer de l'eau en provenance du bassin et est en mesure de fonctionner pour amener cette eau à s'écouler devant la lampe UV, de manière à ce qu'elle soit exposée à la lumière UV en provenance de celle-ci, avant de rejoindre le bassin, **caractérisée en ce que** l'unité formant pompe comporte par ailleurs une sortie de fontaine (90) servant à créer une fontaine.

2. Unité formant pompe pour bassin selon la revendication 1, dans laquelle les composants électriques de la pompe (70) sont contenus dans un contenant étanche (72) qui est étanche contre toute pénétration d'eau et les composants électriques de la lampe sont contenus dans un contenant étanche (82, 86, 87) qui est étanche contre toute pénétration d'eau.

3. Unité formant pompe pour bassin selon la revendication 1, dans laquelle les composants électriques de la pompe (70) et de la lampe UV (80) sont contenus ensemble dans un contenant étanche (72, 82, 86, 87) dans le logement, l'étanchéité du contenant étanche étant contre toute pénétration d'eau.

4. Unité formant pompe pour bassin selon l'une quelconque des revendications précédentes, agencée de telle sorte que la dimension principale de la lampe UV (80) s'étend dans la direction de l'écoulement de l'eau devant celle-ci.

5. Unité formant pompe pour bassin selon l'une quelconque des revendications précédentes, agencée de telle sorte que l'écoulement de l'eau se fait tout autour de la lampe (80) afin de maximaliser l'exposition de l'eau à la lumière UV émise par la lampe.

6. Unité formant pompe pour bassin selon l'une quelconque des revendications précédentes, qui comporte par ailleurs des moyens de filtration mécaniques (46, 50, 55) et qui est agencée de telle sorte que la pompe amène l'eau aspirée en provenance du bassin à passer au travers de ces moyens de telle sorte que l'eau est filtrée par ceux-ci.

7. Unité formant pompe pour bassin selon la revendication 6, dans laquelle les moyens de filtration comportent des ouvertures (46) formées dans le logement (40) de l'unité et au travers desquelles de l'eau en provenance du bassin est aspirée par la pompe.

8. Unité formant pompe pour bassin selon la revendication 6 ou la revendication 7, dans laquelle les moyens de filtration comprennent un élément filtrant (50) qui est en mesure d'être placé de manière amovible dans le logement.

9. Unité formant pompe pour bassin selon l'une quelconque des revendications 6 à 8, qui comporte par ailleurs un dispositif de dérivation (52) permettant à l'eau aspirée en provenance du bassin de contourner au moins une partie des moyens de filtration quand l'écoulement de l'eau au travers de ces moyens est entravé par au moins une obturation partielle de ceux-ci.

10. Unité formant pompe pour bassin selon l'une quelconque des revendications précédentes, qui comporte par ailleurs une structure de filtration biologique (60) agencée avec un rapport surface/volume élevé et agencée dans le logement (40) de telle sorte que l'eau aspirée en provenance du bassin est amenée à s'écouler sur et autour de la structure.

11. Unité formant pompe selon l'une quelconque des revendications précédentes, dans laquelle la sortie de fontaine comporte un tuyau (92) doté d'une partie formant buse (94) au niveau d'une extrémité pour créer la fontaine, dans laquelle la partie formant buse (94) est formée avec des trous au travers de celle-ci agencés pour diriger l'eau pompée au travers de ceux-ci en un jet constitué d'un ou de plusieurs filets d'eau.

12. Unité formant pompe pour bassin selon l'une quelconque des revendications précédentes, qui comporte par ailleurs une sortie de dérivation (100) agencée pour mettre en oeuvre une sortie pour au moins une partie de l'eau aspirée en provenance du bassin, de manière à contourner la sortie de fontaine et à rejoindre le bassin sans former de fontaine.

13. Unité formant pompe pour bassin selon la revendication 12, dans laquelle l'unité est agencée de telle sorte que la sortie de dérivation (100) reçoit l'eau qui s'est déjà écoulée devant la lampe UV (80).

14. Unité formant pompe pour bassin selon la revendication 12 ou la revendication 13, qui comporte par ailleurs un moyen de commande d'écoulement variable servant à commander de manière variable l'écoulement de l'eau au travers de la sortie de dérivation (100) et/ou de la sortie de fontaine (90).

15. Unité formant pompe pour bassin selon la revendication 14, dans laquelle la commande d'écoulement variable est agencée pour être en mesure de fonctionner entre un état dans lequel l'écoulement au travers de la sortie de dérivation (100) est empêché et un état dans lequel toute l'eau aspirée en provenance du bassin peut passer au travers de la sortie de dérivation, et est en mesure de fonctionner dans des états entre ceux-ci.

16. Unité formant pompe pour bassin selon la revendication 14 ou la revendication 15, dans laquelle la commande d'écoulement variable est ajustable par un utilisateur au moyen d'un collier rotatif (102).

17. Unité formant pompe pour bassin selon l'une quelconque des revendications 13 à 16, dans laquelle la sortie de dérivation (100) est positionnée dans l'unité (30) à distance d'une entrée sur celle-ci au travers de laquelle de l'eau est aspirée en provenance du bassin par la pompe électrique.

18. Unité formant pompe pour bassin selon l'une quelconque des revendications précédentes, dans laquelle l'unité comporte une sortie de raccordement (1001) servant à distribuer l'eau par le biais d'un flexible jusqu'à une sortie distante.

19. Unité formant pompe pour bassin selon la revendication 18, dans laquelle l'unité comporte un moyen de commande d'écoulement variable (1102) à des fins de commande variable de l'écoulement de l'eau au travers de la sortie de raccordement.

20. Unité formant pompe pour bassin selon l'une quelconque des revendications 18 à 19, dans laquelle l'unité est agencée de telle sorte que la sortie de raccordement est positionnée dans la trajectoire d'écoulement de telle sorte que l'eau quittant par le biais de la sortie de raccordement aura passé devant la lampe UV.

21. Unité formant pompe pour bassin selon l'une quelconque des revendications précédentes, qui est agencée pour être submersible dans l'eau.

22. Unité formant pompe pour bassin selon l'une quelconque des revendications précédentes, agencée avec une partie formant base (20) et une partie supérieure faisant saillie (30) s'étendant à la verticale depuis celle-ci.

23. Unité formant pompe pour bassin selon l'une quelconque des revendications précédentes, qui est une unité formant pompe pour bassin de jardin privé.
